(19) Europäisches Patentamt
European Patent Office
Office européen des brevets

(11) EP 3 317 251 B1

(12) EUROPÄISCHE PATENTSCHRIFT

(45) Veröffentlichungstag und Bekanntmachung des Hinweises auf die Patenterteilung:
**10.07.2019 Patentblatt 2019/28**

(21) Anmeldenummer: **16727163.4**

(22) Anmeldetag: **31.05.2016**

(51) Int Cl.:
***C07D 211/58*** (2006.01) ***C08K 5/00*** (2006.01)
***C08K 5/3435*** (2006.01)

(86) Internationale Anmeldenummer:
**PCT/EP2016/062301**

(87) Internationale Veröffentlichungsnummer:
**WO 2017/005413 (12.01.2017 Gazette 2017/02)**

(54) **POLYMERADDITIV UND VERFAHREN ZU SEINER HERSTELLUNG**

POLYMER ADDITIVE AND A METHOD FOR THE PRODUCTION THEREOF

ADDITIF POUR POLYMÈRE ET PROCÉDÉ DE PRODUCTION DUDIT ADDITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.07.2015 DE 102015212508**

(43) Veröffentlichungstag der Anmeldung:
**09.05.2018 Patentblatt 2018/19**

(73) Patentinhaber: **Clariant Plastics & Coatings Ltd**
**4132 Muttenz (CH)**

(72) Erfinder:
- **SAHL, Mike**
  **65520 Bad Camberg (DE)**
- **ZEISSBERGER, Eduard**
  **86356 Neusäss (DE)**
- **ZÄH, Matthias**
  **86368 Gersthofen (DE)**
- **SCHACKER, Ottmar**
  **86368 Gersthofen (DE)**
- **STEFFANUT, Pascal**
  **68128 Village-Neuf (FR)**

(74) Vertreter: **Jacobi, Carola**
**Clariant Produkte (Deutschland) GmbH**
**Patent & License Management Chemicals**
**Industriepark Höchst, G 860**
**65926 Frankfurt (DE)**

(56) Entgegenhaltungen:
**WO-A1-2004/016591 CN-A- 103 030 588**
**CN-A- 103 508 938 CN-A- 103 554 009**
**CN-A- 104 557 676 US-A- 5 241 067**

- **CAIRA M R: "CRYSTALLINE POLYMORPHISM OF ORGANIC COMPOUNDS", TOPICS IN CURRENT CHEMISTRY, SPRINGER, BERLIN, DE, Bd. 198, 1. Januar 1998 (1998-01-01), Seiten 163-208, XP001156954, ISSN: 0340-1022, DOI: 10.1007/3-540-69178-2_5**

EP 3 317 251 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft die Herstellung des Polymeradditives der Formel (I) in einer für das Polymeradditiv der Formel (I) bislang unbekannten Kristall-Morphologie sowie dessen Herstellungsprozess.

Das Polymeradditiv der Formel (I):

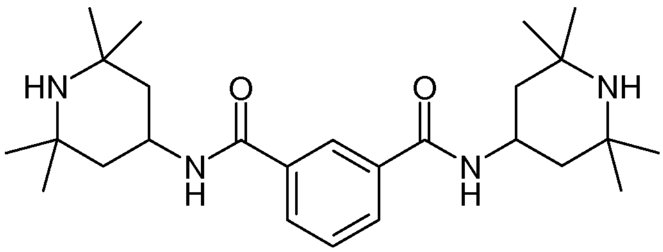

(I)

wurde das erste Mal in der WO 97/43335 beschrieben und wird seitdem insbesondere in Polyamiden als wirkungsvoller Stabilisator gegen den schädlichen Einfluss von Wärme und Licht eingesetzt. Die Herstellung dieser Verbindung erfolgt üblicherweise unter Schotten-Baumann-Bedingungen nach der Reaktionsgleichung (1) gemäß WO 2004/016591:

Reaktionsgleichung (1):

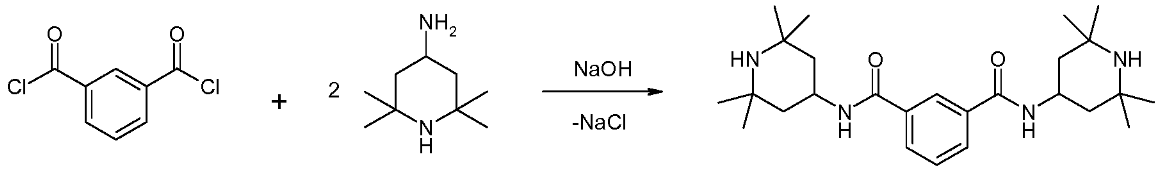


Dabei werden als Lösungsmittel Mischungen aus Wasser und Isopropanol verwendet.

Die Kristallstruktur des Polymeradditives der Formel (I) hergestellt nach Reaktionsgleichung (1) ist im Röntgendiffraktogramm durch sechs stark intensive Peaks bei einem 2-theta-Winkel von 5.7, 14.9, 15.2, 17.8, 22.7, 23.7; vier mittel intensiven Peaks bei einem 2-theta-Winkel von 16.9, 20.2, 21.8, 26.8; und weiteren schwach intensiven Peaks bei einem 2-theta Winkel von 9.3, 10.5, 11.3, 12.9, 14.1, 16.5, 19.7, 24.9, 28.5, 30.7, 31.5, 32.0, 33.0, 34.0, 35.9, 37.6, 39.5, 40.6, 41.1, 43.1, 45.3, 47.2, 48.1, 59.0 charakterisiert. Diese bekannte Kristallphase wird nachstehend als alpha-Phase bezeichnet.

Die alpha-Phase ist in technischem Maßstab schwierig handhabbar, da das aus der Synthese isolierte Pulver staubt, schlecht rieselfähig ist und eine geringe Schüttdichte aufweist, was eine aufwändige Kompaktierung erforderlich macht.

Zusammenfassung der Erfindung:

Eine Aufgabe der vorliegenden Erfindung war es, ein praktikables Verfahren bereitzustellen, mit welchem es möglich ist, das Polymeradditiv der Formel (I) mit verbesserten physikalischen Eigenschaften gegenüber dem Stand der Technik herzustellen.

Eine weitere Aufgabe der Erfindung war es, ein Verfahren zur Herstellung des Polymeradditives der Formel (I) bereitzustellen, das sich dadurch auszeichnet, den Einsatz von toxischen und korrosiven Säurechloriden, die Entstehung von Halogenidsalzen, die eine kostenintensive Abtrennung und Entsorgung erforderlich machen, sowie eine kostenintensive Kühlung zu vermeiden. Zudem soll das Verfahren eine sichere und reproduzierbare Reaktionsführung gewährleisten.

Überraschenderweise wurde gefunden, dass diese Aufgabe durch ein Verfahren gelöst werden kann, das auf einer Amidierung von Isophthalsäurediestern mit 4-Amino-2,2,6,6-tetramethylpiperidin beruht und unter Verwendung eines Katalysators aus der Gruppe der Metallalkoholate (Reaktionsgleichung (2) durchgeführt wird.

Reaktionsgleichung (2):

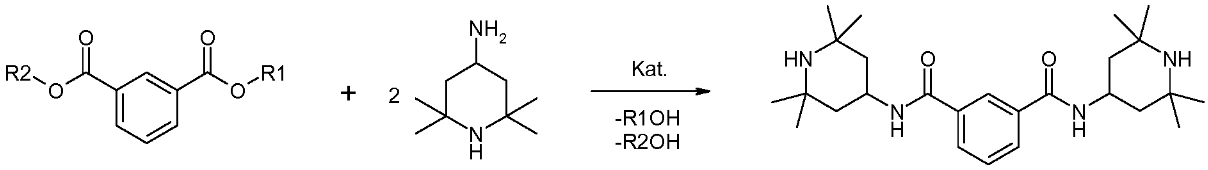


Gegenstand der Erfindung ist ein Verfahren zur Herstellung der Verbindung der Formel (I),

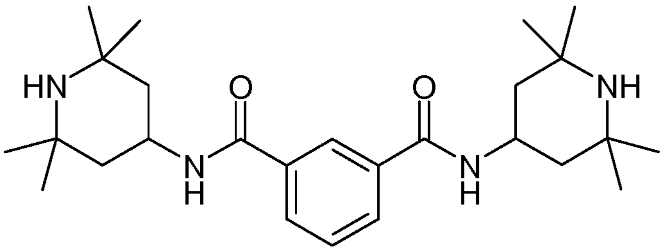

(I)

durch Umsetzung mindestens eines Isophthalsäurediesters der Formel (II)

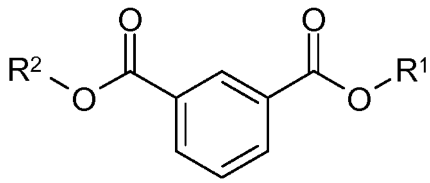

(II)

worin $R^1$ und $R^2$ gleich oder verschieden sind und für einen aliphatischen Rest stehen,
mit 2 Equivalenten 4-Amino-2,2,6,6-tetramethylpiperidin,
in Gegenwart mindestens eines Katalysators aus der Gruppe der Metallalkoholate, bei einer Reaktionstemperatur zwischen 50 und 150 °C.

In einer bevorzugten Form stehen $R^1$ und $R^2$ jeweils für einen aliphatischen Rest, der 1 bis 20, bevorzugt 1 bis 6, besonders bevorzugt 1 bis 4 C-Atome, hat. Der aliphatische Rest kann linear, verzweigt oder zyklisch sein. Er kann gesättigt oder ungesättigt sein, bevorzugt ist er gesättigt. Geeignete aliphatische Reste sind beispielsweise: Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexyl, n-Nonyl, n-Decyl, Cyclopropyl, Cyclobutyl, Cyclopentyl, Cyclohexyl, Cycloheptyl, Cyclooctyl, Cyclononyl, Cyclodecyl. Besonders bevorzugt sind die Reste Methyl, Ethyl, n-Propyl, Isopropyl und n-Butyl.

Besonders bevorzugt sind Isophthalsäurediester, worin die Reste $R^1$ und $R^2$ jeweils Methyl, Ethyl, n-Propyl, Isopropyl oder n-Butyl bedeuten.

Katalysatoren im Sinne der vorliegenden Erfindung sind Alkoholate der allgemeinen Formeln (VI) und/oder (VII)

$X\text{-}OR^{13}$ (VI)

$R^{14}O\text{-}Y\text{-}OR^{15}$ (VII)

wobei die Reste

| | |
|---|---|
| $R^{14}$ und $R^{15}$ | jeweils gleich oder verschieden sind; |
| $R^{13}$, $R^{14}$ und $R^{15}$ | für aliphatische Reste mit jeweils 1 bis 20 C-Atomen stehen, wie z. B. Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexyl, n-Nonyl, n-Decyl, oder $C_3$-$C_{12}$-Cycloalkylreste, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl. |

Bevorzugt sind Methyl, Ethyl und tert.-Butyl.

X steht für Alkalimetalle wie z. B. Lithium, Natrium, Kalium, Rubidium und Cäsium. Bevorzugt sind Lithium, Natrium und Kalium;

Y steht für Erdalkalimetalle wie z. B. Beryllium, Magnesium, Calcium, Strontium und Barium. Bevorzugt sind Magnesium und Calcium.

Bevorzugte Katalysatoren sind beispielsweiseNa-methanolat, K-methanolat, Li-methanolat, Na-ethanolat, K-ethanolat, Li-ethanolat, Na-n-propanolat, K-n-propanolat,Na-isopropanolat, K-isopropanolat, Na-butylat, K-butylat, Na-isobutylat, K-isobutylat, Na-sec.-butylat, K-sec.-butylat, Na-tert.-amylat, Na-amylat, K-amylat, K-tert.-butylat.

Der Katalysator wird zweckmäßigerweise in Mengen von 1 bis 20 mol-%, bevorzugt 2 bis 15 mol-%, insbesondere 3 bis 12 mol-%, bezogen auf den Isophthalsäurediester, dem Reaktionsgemisch hinzugefügt.

Um Einbußen an Ausbeute und Reinheit zu verhindern, ist es weiterhin zweckmäßig, dass der Katalysator bei der Zugabe in das Reaktionsgemisch vollständig in einem Alkohol, beispielsweise in dem Alkohol, aus dem er hergestellt wurde, gelöst ist, vorzugsweise in einer 1 bis 4-fachen Menge Alkohol, bezogen auf die Masse des Katalysators.

In einer bevorzugten Ausführungsform des erfindungsgemäßen Verfahrens (Verfahrensvariante a) wird die Umsetzung lösungsmittelfrei und in einer Reaktionsapparatur aus der Gruppe der Mischer, Kneter und Extruder durchgeführt. In diesen Reaktionsapparaturen können flüssige und feste Mischungen aus flüssigen und festen Stoffen unter Verwendung von Scherkräften durchmischt werden.

Im Sinne der vorliegenden Erfindung wird unter lösungsmittelfreiem Verfahren ein Verfahren verstanden, das kein oder nur so wenig eines organischen Lösungsmittels, z. B. Alkohole, aliphatische oder aromatische Kohlenwasserstoffe, enthält, dass das Reaktionsprodukt unter den gegebenen Reaktionstemperaturen nicht in Lösung geht und aus dem Reaktionsgemisch direkt als Feststoff ausfällt. Bei Verfahrensvariante a) ist es zweckmäßig, dass der Isophthalsäurediester mit etwa zwei Equivalenten 4-Amino-2,2,6,6-tetramethylpiperidin in der Reaktionsapparatur vermischt wird, d. h. weder der Isophthalsäurediester noch das Amin werden im Überschuss eingesetzt. Die durch den Katalysator in das Reaktionsgemisch eingeführte Menge an Alkohol ist bezüglich der Masse der Reaktionspartner so klein, dass die erfindungsgemäße Umsetzung als lösungsmittelfrei bezeichnet werden kann. Im Allgemeinen wird der durch den Katalysator eingeführte Alkohol zusammen mit dem bei der Reaktion entstehenden Alkohol aus dem Reaktionsgemisch entfernt, z. B. destillativ.

Das gewünschte Reaktionsprodukt der Formel (I) ist bei der erfindungsgemäßen Reaktionstemperatur fest (Schmelzpunkt ca. 272 °C) und fällt als Feststoff an, ohne vorher eine Schmelze durchlaufen zu haben.

Als Reaktionsapparatur wird zweckmäßigerweise eine Vorrichtung, mit der flüssige und feste Mischungen aus flüssigen und festen Stoffen unter Verwendung von Scherkräften durchmischt werden können, verwendet. In einem statischen Gehäuse werden die Reaktionsmassebewegungen durch interne mechanische Rühr- oder Mischvorrichtungen bewirkt. Eine Drehung des Gehäuses und statische mechanische Einbauten sind auch möglich, aber nicht bevorzugt. Eine Zugabe von Mahlkörpern wie Kugeln aus Stahl oder aus verschiedenen Keramiken ist möglich, aber nicht notwendig. Die Reaktionsapparatur kann im Chargenbetrieb oder im Dauerbetrieb betrieben werden.

Die Reaktionsapparatur bei Verfahrensvariante a) ist zweckmäßigerweise ein Kneter oder Mischer, der vorzugsweise mit Sigmaschaufeln, Mastikatorschaufeln, Pflugscharen, Beckerschaufeln oder Wurfpaddeln ausgerüstet ist. Alternativ kann die Reaktionsapparatur auch ein Extruder sein, z. B. ein einspindeliger oder zweispindeliger Schneckenkneter.

In Frage kommende Apparaturen sind unter anderem:

- Planetenmischer, welche mit Mischwerkzeugen wie z. B. Kreuzbalken, einem Flügelmischwerk, einem Wendelmischwerk oder einem Kammschaufelrührwerk ausgestattet sind,
- horizontal oder vertikal arbeitende Schaufelkneter, welche mit Mischwerkzeugen, wie z. B. Sigmaschaufeln, Mastikatorschaufeln, Pflugschare, Beckerschaufeln oder Wurfpaddeln ausgestattet sind,
- einspindelige Schneckenkneter,
- zweispindelige Schneckenkneter, ausgerüstet mit gegenläufigen oder gleichläufigen Schneckenbändern,
- Bandschneckenmischer, welche mit einer Doppelbandschnecke ausgestattet sind,
- Konusmischer mit Ein- oder Zweiwendelausführung,
- Taumelmischer,
- Freifallmischer, dessen Trommelwände optional mit Mischwerkzeugen, wie z. B. Spiralen, Paddeln oder Schaufeln ausgestattet sind,
- horizontal oder vertikal arbeitende Zwangsmischer, welche mit Mischwerkzeugen, wie z. B. Sigmaschaufeln, Mastikatorschaufeln, Pflugschare, Beckerschaufeln oder Wurfpaddeln in Kombination mit einem Schneidrotor ausgestattet sind,

bevorzugt horizontal arbeitende Schaufelkneter, welche mit Mischwerkzeugen oder Kombinationen aus Mischwerkzeugen, wie z. B. Sigmaschaufeln, Mastikatorschaufeln, Pflugscharen, Beckerschaufeln oder Wurfpaddeln ausgestattet sind,
weiterhin bevorzugt zweispindelige Schneckenkneter mit gegenläufigen Schneckenbändern,
weiterhin bevorzugt horizontal arbeitende Zwangsmischer, welche mit Mischwerkzeugen oder Kombinationen aus Mischwerkzeugen, wie z. B. Sigmaschaufeln, Mastikatorschaufeln, Pflugschare, Beckerschaufeln oder Wurfpaddeln ausgestattet sind in Kombination mit einem in der Trommel installierten Schneidrotor;

- besonders bevorzugt horizontal arbeitende Zwangsmischer, welche bei einer Froudezahl zwischen 0,1 und 6, bevorzugt zwischen 0,25 und 5, besonders bevorzugt zwischen 0,4 und 4, arbeiten und mit Mischwerkzeugen oder Kombinationen aus Mischwerkzeugen wie z. B. Sigmaschaufeln, Mastikatorschaufeln, Pflugschare, Beckerschaufeln oder Wurfpaddeln ausgestattet sind in Kombination mit einem in der Trommel installierten Schneidrotor.

Der Bewegungszustand insbesondere von Feststoffmischungen bei horizontalen Zwangsmischern lässt sich durch die dimensionslose Froude-Zahl Fr charakterisieren. Dabei gilt:

$$Fr = v^2 / r \cdot g$$

mit

v = Umfangsgeschwindigkeit der Mischelemente
r = Radius der Mischtrommel
g = Erdbeschleunigung.

In einer speziellen Ausführungsform von Verfahrensvariante a) werden 1 Äquivalent Isophthalsäurediester und 2 Äquivalente 4-Amino-2,2,6,6-tetramethylpiperidin bei einer Temperatur zwischen 40 und 150 °C, bevorzugt 45 bis 120 °C, insbesondere 50 bis 110 °C, zweckmäßigerweise unter Schutzgas, zu einer homogenen (einphasigen) Mischung ohne Lösungsmittel und vorzugsweise in einem horizontal arbeitenden Zwangsmischer, welcher bei einer Froudezahl zwischen 0,1 und 6, bevorzugt zwischen 0,25 und 5 besonders bevorzugt zwischen 0,4 und 4 arbeitet und mit Mischwerkzeugen oder Kombinationen aus Mischwerkzeugen wie z. B. Sigmaschaufeln, Mastikatorschaufeln, Pflugschare, Beckerschaufeln oder Wurfpaddeln ausgestattet ist, optional in Kombination mit einem in der Trommel installierten Schneidrotor, miteinander vermischt. Als Schutzgas besonders bevorzugt sind Stickstoff und Argon. Nach Entstehung der homogenen Mischung wird die Reaktionsmischung auf die vorgesehene Temperatur geregelt, die zwischen 50 und 150 °C, bevorzugt 80 bis 120 °C, speziell 90 bis 110 °C liegt. Anschließend wird der Katalysator in Mengen von 1 bis 20 mol-%, bevorzugt 2 bis 15 mol-%, insbesondere 3 bis 12 mol-%, bezüglich des Isophthalsäurediesters in das homogene Reaktionsgemisch hineingegeben. Das Reaktionsgemisch wird nach der Zugabe des Katalysators zweckmäßigerweise für einen Zeitraum von 10 bis 400 Minuten, bevorzugt 30 bis 300 Minuten, insbesondere 45 bis 120 Minuten, durch die Rühr- und Mischvorrichtungen der Reaktionsapparatur durchmischt und der entstehende Alkohol bei einem Druck von 0,1 bis 1013 mbar, bevorzugt 0,2 bis 500 mbar, insbesondere 0,3 bis 250 mbar, aus dem Reaktionsgemisch entfernt, vorzugsweise destillativ. Nach Belüften der Reaktionsapparatur, bevorzugt mit Schutzgas, wird das Reaktionsgemisch auf Raumtemperatur abgekühlt. Das Polymeradditiv der Formel (I) fällt dabei als Feststoff in hoher Reinheit und hohen Ausbeuten an.

In einer weiteren Ausführungsform des erfindungsgemäßen Verfahrens (Verfahrensvariante b) wird der Isophthalsäurediester in einem Überschuss 4-Amino-2,2,6,6-tetramethylpiperidin gelöst. Das molare Verhältnis zwischen Isophthalsäurediester und 4-Amino-2,2,6,6-tetramethylpiperidin beträgt in diesem Fall 1 zu 3,9 mol bis 1 zu 27,5 mol, bevorzugt 1 zu 6,25 mol bis 1 zu 18 mol, besonders bevorzugt 1 zu 8,6 mol bis 1 zu 13,35 mol. Anschließend wird der Katalysator vorzugsweise bei einer Temperatur zwischen 50 und 80°C, insbesondere zwischen 60 und 70 °C, hinzugegeben und die Reaktionsmischung vorzugsweise auf eine Temperatur zwischen 80 und 150 °C, insbesondere zwischen 85 und 130 °C, erhitzt. Optional kann der entstehende Alkohol während der Reaktion aus dem Reaktionsgemisch destillativ entfernt werden. Das Polymeradditiv der Formel (I) fällt aus dem Reaktionsgemisch aus und kann abfiltriert werden. Die Verfahrensvariante b) wird zweckmäßigerweise wie die Variante a) lösungsmittelfrei durchgeführt.

In einer weiteren Ausführungsform des erfindungsgemäßen Verfahrens (Verfahrensvariante c) wird der Isophthalsäurediester mit etwa zwei Equivalenten 4-Amino-2,2,6,6-tetramethylpiperidin und einem lipophilen flüssigen Kohlenwasserstoff oder einer Mischung solcher Kohlenwasserstoffe, z. B. Hexan, Heptan, Octan, Dodecan, Petrolether, Shellsol, White Spirit oder Exxsol gelöst, vorzugsweise bei einer Temperatur zwischen 20 und 40 °C, anschließend der Katalysator vorzugsweise bei einer Temperatur zwischen 50 und 80 °C, insbesondere zwischen 60 und 70 °C, hinzugegeben. Das Massenverhältnis zischen Lösungsmittel und Edukte liegt zwischen 90 zu 10 Gew.-% bis 50 zu 50 Gew.-%, bevorzugt 85 zu 15 Gew.-% bis 60 zu 40 Gew.-%, besonders bevorzugt 80 zu 20 Gew.-% bis 70 zu 30 Gew.-%

Anschließend wird die Reaktionsmischung vorzugsweise auf eine Temperatur zwischen 80 und 150 °C, insbesondere zwischen 85 und 110 °C, erhitzt. Optional kann der entstehende Alkohol aus dem Reaktionsgemisch destillativ entfernt werden. Das Polymeradditiv der Formel (I) fällt aus dem Reaktionsgemisch aus und kann abfiltriert werden.

Die Vorteile des erfindungsgemäßen Verfahrens gegenüber dem Stand der Technik bestehen unter anderem darin, dass das Polymeradditiv der Formel (I) lösungsmittelfrei (Varianten a und b), ohne Verwendung von Eduktüberschüssen (Varianten a und c), in hoher Reinheit und nahezu quantitativen Ausbeuten, und ohne zusätzliche Aufreinigungsschritte synthetisiert werden kann.

Des Weiteren vermeidet das erfindungsgemäße Verfahren die Entstehung von mit Halogenidsalzen oder gegebenenfalls Edukt belastetem Abwasser, dessen vorschriftsmäßige Entsorgung einen signifikanten Einfluss auf die Gesamtkosten der Herstellung des Polymeradditives der Formel (I) hat. Die Vermeidung von Halogenidsalzen in Form von Nebenkondensationsprodukten schließt weiterhin die Kontamination des Polymeradditives der Formel (I) mit diesen aus, was wiederum für dessen kommerzielle Verbreitung von entscheidender Bedeutung sein kann.

Die besonders bevorzugte Variante ist Verfahrensvariante a).

Des Weiteren wurde überraschenderweise gefunden, dass das erfindungsgemäße Verfahren, insbesondere in den Verfahrensvarianten a), b) und c), die Verbindung der Formel (I) in einer neuen Kristallmodifikation liefert, welche beta-Phase genannt wird. Die beta-Phase ist im Röntgendiffraktogramm durch zwei stark intensive Peaks bei einem 2-theta-Winkel von 15.0, 22.7; sechs mittel intensiven Peaks bei einem 2-theta-Winkel von 5.0, 11.3, 18.9, 20.8, 21.6, 23.6; und weiteren zahlreichen schwach intensiven Peaks beispielsweise bei einem 2-theta Winkel von 7.1, 10.1, 12.2, 16.0, 16.8, 17.8, 18.2, 19.7, 24.8, 25.6, 26.7, 27.2, 28.5, 29.3, 30.5, 32.5, 33.5, 34.1, 36.2, 37.3, 38.4, 40.6, 41.3, 44.3, 46.5 charakterisiert. Peaks mit starker Intensität sind solche, die eine relative Intensität von über 50 % haben. Peaks mit mittlerer Intensität sind solche, die eine relative Intensität von 35 bis 50 % haben. Peaks unter 35 % werden als schwach intensiv bezeichnet.

Gegenstand der Erfindung ist weiterhin eine Verbindung der Formel (I) erhältlich nach dem vorstehend beschriebenen erfindungsgemäßen Verfahren.

Gegenstand der Erfindung ist weiterhin eine Verbindung der Formel (I) in der beta-Phase

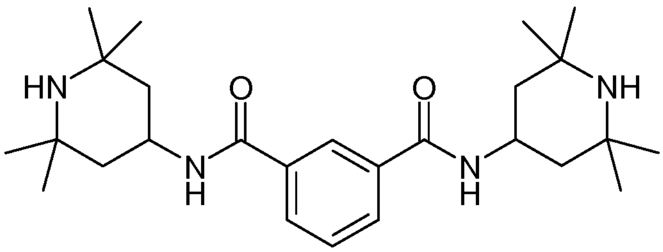

(I)

gekennzeichnet durch die charakteristischen Signale im Röntgenpulverdiagramm, gemessen mit Cu $K_{alpha}$-Strahlung (0,154 nm), bei einem 2-Theta-Winkel, von 15.0 und 22.7, die eine starke Intensität haben, und von 5.0, 11.3, 18.9, 20.8, 21.6 und 23.6, die eine mittlere Intensität haben.

Die Verbindung der Formel (I) in der beta-Phase ist überraschenderweise durch vorteilhafte physikalische Eigenschaften gekennzeichnet, wie z. B. eine geringe Staubbildung, bessere Rieselfähigkeit und höhere Schüttdichte im Vergleich zur bislang bekannten alpha-Phase. Dadurch wird eine Kompaktierung wesentlich vereinfacht oder überflüssig. Die Schüttdichte der beta-Phase, erhalten aus dem erfindungsgemäßen Verfahren, liegt im Bereich von 0,40 bis 0,65 g/cm$^3$, vorzugsweise von 0,45 und 0,60 g/cm$^3$.

Die Bestimmung der Schüttdichte kann nach DIN 53468, EN ISO 60 bei Raumtemperatur (23 - 25 °C) erfolgen, z. B. mit Hilfe eines Karg Fluometer ADP. Die Verbindung der Formel (I) in der beta-Phase ist als Polymeradditiv verwendbar, insbesondere zur Stabilisierung insbesondere von Polymeren, vorzugsweise Polyamiden, EVOH und Polyestern, z. B. PET, gegen Licht und Wärme sowie zur Verbesserung der Sauerstoff-Barriereeigenschaften, insbesondere von EVOH.

Beispiele:

Die Analytik der Produkte erfolgte mittels $^1$H-NMR-Spektroskopie bei 400 MHz in DMSO-$d_6$ und HPLC unter isokratischen Bedingungen. Als kontinuierliche Phase wurde bei der HPLC ein Acetonitril/Wasser-Gemisch (70 Gew.-% / 30 Gew.-%) verwendet. Als stationäre Phase wurde eine RP-18-Säule verwendet.

Die Röntgendiffraktogramme sind mittels Cu K alpha-Strahlung (0,154 nm) bei einer Temperatur von 20 °C auf einem PANalytical X'Pert Pro MPD PW 3040/00 mit einem X'Celerator Detektor gemessen worden.

Herstellung von N,N'-Bis-(2,2,6,6-tetramethyl-piperidin-4-yl)-isophthalamid in der β Phase (Smp.: 272 °C):

Beispiel 1 (Verfahrensvariante a):

In einem horizontal arbeitenden Zwangsmischer, welcher bei einer Froudezahl von 2,16 arbeitet, und mit Pflugscharen, einer Destillationskolonne und einem Schutzgasanschluss ausgestattet ist, werden 2,5 mol Dimethylisophthalat (Smp. 64 - 66 °C) und 5 mol 4-Amino-2,2,6,6-tetramethylpiperidin (flüssig bei Raumtemperatur) bei einer Temperatur von 60 °C unter Stickstoff miteinander homogenisiert, bis eine einphasige flüssige Mischung entsteht. Nach der Zugabe von 59,4 g einer Natriummethanolatlösung (25 gew.-%ig in Methanol) wird die Reaktionsmasse für eine Dauer von 90 Minuten bei 110 °C durchmischt. Der Alkohol aus der Katalysatorzubereitung und der während der Reaktion entstehende Alkohol werden aus dem Zwangsmischer destillativ entfernt. Nach dem Austragen des Feststoffs und Trocknung bis zur Gewichtskonstanz wurden 1088,4 g (Massenausbeute: 98,5 %) eines weißen Pulvers isoliert.

Die Schüttdichte des Produkts betrug ca. 0,53 g/cm$^3$.

Eine HPLC-Analyse des weißen Feststoffes ergab folgende Zusammensetzung:

| Stoff | mol-% |
|---|---|
| Produkt | 99,7 |
| Monoamid | 0,1 |
| 4-Amino-2,2,6,6-tetramethypiperidin | 0,1 |
| Dimethylisophthalat | 0,1 |

Im Röntgendiffraktogramm wurden für das Produkt folgende Signale identifiziert:

| 2 theta | d/Angström | Absolute Intensität | Relative Intensität/% |
|---|---|---|---|
| 4,99 | 17,69 | 10429,00 | 42,28 |
| 7,12 | 12,42 | 8059,00 | 32,67 |
| 10,11 | 8,75 | 3057,00 | 12,39 |
| 11,27 | 7,85 | 11217,00 | 45,47 |
| 12,19 | 7,26 | 4110,00 | 16,66 |
| 14,97 | 5,92 | 24667,00 | 100,00 |
| 16,00 | 5,54 | 5368,00 | 21,76 |
| 16,83 | 5,27 | 3289,00 | 13,33 |
| 17,76 | 4,99 | 6477,00 | 26,26 |
| 18,22 | 4,87 | 7616,00 | 30,88 |
| 18,88 | 4,70 | 9158,00 | 37,13 |
| 19,68 | 4,51 | 7802,00 | 31,63 |
| 20,82 | 4,27 | 11672,00 | 47,32 |
| 21,57 | 4,12 | 11646,00 | 47,21 |
| 22,67 | 3,92 | 17291,00 | 70,10 |
| 23,56 | 3,78 | 9127,00 | 37,00 |
| 24,78 | 3,59 | 5763,00 | 23,36 |
| 25,60 | 3,48 | 4740,00 | 19,22 |
| 26,70 | 3,34 | 5518,00 | 22,37 |
| 27,21 | 3,28 | 5428,00 | 22,01 |
| 28,52 | 3,13 | 4108,00 | 16,65 |
| 29,29 | 3,05 | 4554,00 | 18,46 |

(fortgesetzt)

| 2 theta | d/Angström | Absolute Intensität | Relative Intensität/% |
|---|---|---|---|
| 30,48 | 2,93 | 5588,00 | 22,65 |
| 32,45 | 2,76 | 3628,00 | 14,71 |
| 33,45 | 2,68 | 4144,00 | 16,80 |
| 34,08 | 2,63 | 4649,00 | 18,85 |
| 36,22 | 2,48 | 4644,00 | 18,83 |
| 37,33 | 2,41 | 4463,00 | 18,09 |
| 38,37 | 2,35 | 4433,00 | 17,97 |
| 40,61 | 2,22 | 5763,00 | 23,36 |
| 41,32 | 2,18 | 6110,00 | 24,77 |
| 44,33 | 2,04 | 6077,00 | 24,64 |
| 46,49 | 1,95 | 5742,00 | 23,28 |

Beispiel 2 (Verfahrensvariante a):

In einem horizontal arbeitenden Zwangsmischer, welcher bei einer Froudezahl von 2,16 arbeitet, und mit Pflugscharen, einer Destillationskolonne und einem Schutzgasanschluss ausgestattet ist, werden 2,5 mol Dibutylisophthalat (flüssig bei Raumtemperatur) und 5 mol 4-Amino-2,2,6,6-tetramethylpiperidin (flüssig bei Raumtemperatur) bei einer Temperatur von 60 °C unter Stickstoff miteinander homogenisiert. Nach der Zugabe von 59,4 g einer Natriummethanolatlösung (25 gew.-%ig in Methanol) wird die Reaktionsmasse für eine Dauer von 180 Minuten bei 130 °C durchmischt. Zusätzlich wird ein Vakuum von 50 mbar angelegt. Der Alkohol aus der Katalysatorzubereitung und der während der Reaktion entstehende Alkohol werden aus dem Zwangsmischer destillativ entfernt. Nach dem Austragen des Feststoffs und Trocknung bis zur Gewichtskonstanz wurden 1075,6 g (Massenausbeute: 97,3 %) eines weißen Pulvers isoliert.
Die Schüttdichte des Produkts betrug ca. 0,53 g/cm$^3$.

Eine HPLC-Analyse des weißen Feststoffes ergab folgende Zusammensetzung:

| Stoff | mol-% |
|---|---|
| Produkt | 99,7 |
| Monoamid | 0,1 |
| 4-Amino-2,2,6,6-tetramethypiperidin | 0,1 |
| Dimethylisophthalat | 0,1 |

Im Röntgendiffraktogramm wurden für das Produkt dieselben charakteristischen Signale wie in Beispiel 1 angegeben beobachtet.

Beispiel 3 (Verfahrensvariante b):

In einem Mehrhalskolben mit Rückflusskühler, KPG-Rührer, $N_2$-Einlass und Innenthermometer löst man bei einer Temperatur von 30 °C 1 mol Isophthalsäuredimethylester in 8,6 mol 4-Amino-2,2,6,6-tetramethylpiperidin. Anschließend wird das Reaktionsgemisch auf eine Temperatur von 50 °C erhitzt und 23,76 g Natriummethanolat, gelöst in Methanol (25 gew.-%ig), hinzugegeben. Nach der Zugabe des Katalysators wird das Reaktionsgemisch für 4 Stunden auf eine Temperatur von 90°C erhitzt. Das Polymeradditiv der Formel (I) fällt während dieser Zeit aus dem Reaktionsgemisch aus und wird nach Ablauf von 4 Stunden bei Raumtemperatur abgesaugt. Nach Trocknung bis zur Gewichtskonstanz konnten 415 g eines weißen Feststoffs (94 % Ausbeute) isoliert werden.

Eine HPLC-Analyse des weißen Feststoffes ergab folgende Zusammensetzung:

| Stoff | mol-% |
|---|---|
| Produkt | >99,7 |
| Monoamid | <0,1 |
| 4-Amino-2,2,6,6-tetramethypiperidin | <0,1 |
| Dimethylisophthalat | <0,1 |

Im Röntgendiffraktogramm wurden für das Produkt dieselben charakteristischen Signale wie in Beispiel 1 angegeben beobachtet. Die Schüttdichte des Produkts betrug 0,54g/cm$^3$.

Beispiel 4 (Verfahrensvariante c):

In einem 4l Mehrhalskolben mit Rückflusskühler, KPG-Rührer, $N_2$-Einlass und Innenthermometer löst man bei einer Temperatur von 40 °C 1 mol Isophthalsäuredimethylester und 2 mol 4-Amino-2,2,6,6-tetramethylpiperidin in 1700 g n-Heptan. Anschließend wird das Reaktionsgemisch auf eine Temperatur von 50 °C erhitzt und 23,76 g Natriummethanolat, gelöst in Methanol (25 gew.-%ig) hinzugegeben. Nach der Zugabe des Katalysators wird das Reaktionsgemisch für 6 Stunden auf eine Temperatur von 90 °C erhitzt. Das Polymeradditiv der Formel (I) fällt während dieser Zeit aus dem Reaktionsgemisch aus und wird nach Ablauf von 6 Stunden bei Raumtemperatur abgesaugt. Nach Trocknung bis zur Gewichtskonstanz konnten 424 g eines weißen Feststoffs (96 % Ausbeute) isoliert werden.

Eine HPLC-Analyse des weißen Feststoffes ergab folgende Zusammensetzung:

| Stoff | mol-% |
|---|---|
| Produkt | >99,5 |
| Monoamid | 0,2 |
| 4-Amino-2,2,6,6-tetramethypiperidin | 0,2 |
| Dimethylisophthalat | <0,1 |

Im Röntgendiffraktogramm wurden für das Produkt dieselben charakteristischen Signale wie in Beispiel 1 angegeben beobachtet. Die Schüttdichte des Produkts betrug 0,57 g/cm$^3$.

Beispiel 5 (Vergleichsbeispiel):

Herstellung von N,N'-Bis-(2,2,6,6-tetramethyl-piperidin-4-yl)-isophthalamid in der $\alpha$-Phase gemäß DE 60315329 T2 (Smp.: 272 °C):
In einen Reaktionskolben werden 0,95 mol (150,5 g) 4-Amino-2,2,6,6-tetramethylpiperidin und 1,07 mol (85,2 g) 50 gew.-%ige wässrige NaOH-Lösung zu 470 g Isopropanol und 260 g entmineralisiertem Wasser gegeben. Unter Rühren werden 0,5 mol (102,1 g) Isophthalsäuredichlorid zugegeben. Anschließend wird die Reaktionsmischung auf eine Temperatur von 100 °C erhitzt, wobei der Feststoff vollständig in Lösung geht. Es bilden sich 2 flüssige Phasen, von denen die organische Phase abgetrennt, mit Wasser versetzt und der Alkohol abdestilliert wird. Nach Abkühlung wird das Produkt abfiltriert, mit Wasser gewaschen und bis zur Gewichtskonstanz getrocknet. Die Ausbeute des Reaktionsproduktes beträgt 200,0 g (95 % des theoretischen Werts)

Die Schüttdichte des Produkts beträgt ca. 0,20 g/cm$^3$.

Im Röntgendiffraktogramm wurden folgende Signale identifiziert:

| 2 theta | d/Angström | Absolut Intensität | Relative Intensität/% |
|---|---|---|---|
| 5,66 | 15,62 | 31473,00 | 100,00 |
| 9,33 | 9,48 | 7325,00 | 23,27 |
| 10,54 | 8,40 | 3475,00 | 11,04 |
| 11,28 | 7,84 | 7997,00 | 25,41 |
| 12,87 | 6,88 | 5399,00 | 17,15 |

(fortgesetzt)

| 2 theta | d/Angström | Absolut Intensität | Relative Intensität/% |
|---|---|---|---|
| 14,14 | 6,26 | 5640,00 | 17,92 |
| 14,89 | 5,95 | 25649,00 | 81,50 |
| 15,16 | 5,84 | 28327,00 | 90,00 |
| 16,50 | 5,37 | 7164,00 | 22,76 |
| 16,91 | 5,24 | 13596,00 | 43,20 |
| 17,78 | 4,99 | 16524,00 | 52,50 |
| 19,67 | 4,51 | 10433,00 | 33,15 |
| 20,19 | 4,40 | 12160,00 | 38,64 |
| 21,79 | 4,08 | 11911,00 | 37,85 |
| 22,66 | 3,92 | 21629,00 | 68,72 |
| 23,68 | 3,76 | 17097,00 | 54,32 |
| 24,87 | 3,58 | 7694,00 | 24,45 |
| 26,77 | 3,33 | 10583,00 | 33,63 |
| 28,45 | 3,14 | 6061,00 | 19,26 |
| 30,72 | 2,91 | 6330,00 | 20,11 |
| 31,45 | 2,84 | 4019,00 | 12,77 |
| 32,02 | 2,79 | 3494,00 | 11,10 |
| 32,98 | 2,72 | 6001,00 | 19,07 |
| 34,03 | 2,63 | 5111,00 | 16,24 |
| 35,90 | 2,50 | 4840,00 | 15,38 |
| 37,62 | 2,39 | 4396,00 | 13,97 |
| 39,48 | 2,28 | 6330,00 | 20,11 |
| 40,61 | 2,22 | 6176,00 | 19,62 |
| 41,05 | 2,20 | 6250,00 | 19,86 |
| 43,13 | 2,10 | 9371,00 | 29,77 |
| 45,28 | 2,00 | 5454,00 | 17,33 |
| 47,17 | 1,93 | 5973,00 | 18,98 |
| 48,14 | 1,89 | 5824,00 | 18,50 |
| 58,99 | 1,57 | 4621,00 | 14,68 |

**Patentansprüche**

**1.** Verbindung der Formel (I)

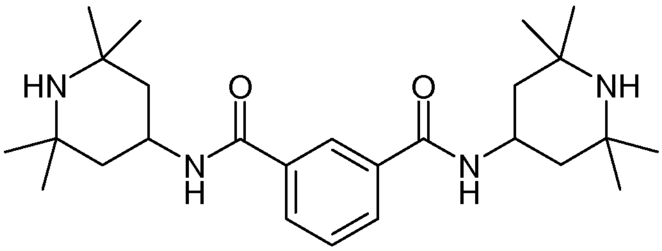

(I)

**gekennzeichnet durch** die charakteristischen Signale im Röntgenpulverdiagramm, gemessen mit Cu $K_{alpha}$-Strahlung bei einem 2-Theta-Winkel von 15.0 und 22.7, die eine starke Intensität haben, und von 5.0, 11.3, 18.9, 20.8, 21.6 und 23.6, die eine mittlere Intensität haben.

2. Verfahren zur Herstellung der Verbindung der Formel (I) nach Anspruch 1, durch Umsetzung mindestens eines Isophthalsäurediesters der Formel (II)

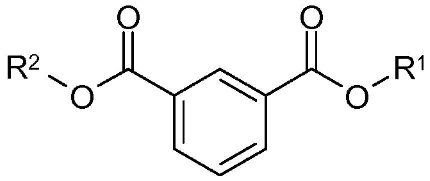

(II)

worin $R^1$ und $R^2$ gleich oder verschieden sind und für einen aliphatischen Rest stehen,
mit 2 Equivalenten 4-Amino-2,2,6,6-tetramethylpiperidin in Gegenwart mindestens eines Katalysators aus der Gruppe der Metallalkoholate und bei einer Reaktionstemperatur zwischen 50 und 150 °C.

3. Verfahren nach Anspruch 2, **dadurch gekennzeichnet, dass** das Metallalkoholat eine Verbindung der allgemeinen Formel (VI) und/oder (VII) ist

$X\text{-}OR^{13}$ (VI)

$R^{14}O\text{-}Y\text{-}OR^{15}$ (VII)

wobei

$R^{14}$ und $R^{15}$ jeweils gleich oder verschieden sind und
$R^{13}$, $R^{14}$ und $R^{15}$ für aliphatische Reste mit jeweils 1 bis 20 C-Atomen stehen,
X für ein Alkalimetall, und
Y für ein Erdalkalimetall stehen.

4. Verfahren nach Anspruch 2 oder 3, **dadurch gekennzeichnet, dass** das Metallalkoholat Na-methanolat, K-methanolat, Li-methanolat, Na-ethanolat, K-ethanolat, Li-ethanolat, Na-n-propanolat, K-n-propanolat, Na-isopropanolat, K-isopropanolat, Na-butylat, K-butylat, Na-isobutylat, K-isobutylat, Na-sec.-butylat, K-sec.-butylat, Na-tert.-amylat, Na-amylat, K-amylat und/oder K-tert.-butylat ist.

5. Verfahren nach einem oder mehreren der Ansprüche 2 bis 4, **dadurch gekennzeichnet, dass** das Metallalkoholat in Mengen von 1 bis 20 mol-%, bezogen auf Isophthalsäurediester, dem Reaktionsgemisch hinzugefügt wird.

6. Verfahren nach einem oder mehreren der Ansprüche 2 bis 5, **dadurch gekennzeichnet, dass** die Umsetzung lösungsmittelfrei und in einer Reaktionsapparatur aus der Gruppe der Mischer, Kneter und Extruder durchgeführt wird.

7. Verfahren nach Anspruch 6, **dadurch gekennzeichnet, dass** das Reaktionsgemisch nach der Zugabe des Katalysators für einen Zeitraum von 10 bis 400 Minuten bei der Reaktionstemperatur durch den Kneter, Mischer oder Extruder durchmischt wird.

**8.** Verfahren nach Anspruch 6 oder 7, **dadurch gekennzeichnet, dass** der Kneter oder Mischer mit Sigmaschaufeln, Mastikatorschaufeln, Pflugscharen, Beckerschaufeln und/oder Wurfpaddeln ausgerüstet ist.

**9.** Verfahren nach mindestens einem der Ansprüche 2 bis 5, **dadurch gekennzeichnet, dass** der Isophthalsäurediester in einem Überschuss an 4-Amino-2,2,6,6-tetramethylpiperidin gelöst wird.

**10.** Verfahren nach mindestens einem der Ansprüche 2 bis 5, **dadurch gekennzeichnet, dass** der Isophthalsäurediester mit etwa zwei Equivalenten 4-Amino-2,2,6,6-tetramethylpiperidin und einem lipophilen flüssigen Kohlenwasserstoff oder einer Mischung solcher Kohlenwasserstoffe gelöst wird.

**11.** Verfahren nach einem oder mehreren der Ansprüche 2 bis 10, **dadurch gekennzeichnet, dass** der bei der Reaktion entstehende Alkohol aus dem Reaktionsgemisch entfernt wird.

**12.** Verfahren nach einem oder mehreren der Ansprüche 2 bis 11, **dadurch gekennzeichnet, dass** das Reaktionsprodukt der Formel (I) aus dem Reaktionsgemisch als Feststoff ausfällt.

**13.** Verwendung einer Verbindung der Formel (I) gemäß Anspruch 1 als Polymeradditiv.

**14.** Verwendung nach Anspruch 13 zur Stabilisierung von Polymeren gegen Licht und Wärme und zur Verbesserung der Sauerstoff-Barriereeigenschaften von Polymeren.

**15.** Verwendung nach Anspruch 13 oder 14, **dadurch gekennzeichnet, dass** die Polymere Polyamide, Polyester oder EVOH sind.

## Claims

**1.** Compound of the formula (I)

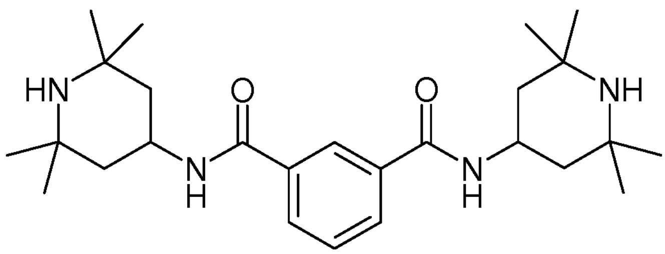

(I)

**characterized by** the characteristic signals in the x-ray powder diagram, measured with Cu $K_{alpha}$ radiation at a 2-theta angle of 15.0 and 22.7 having a high intensity and of 5.0, 11.3, 18.9, 20.8, 21.6 and 23.6 having a moderate intensity.

**2.** Process for preparing the compound of the formula (I) according to Claim 1, by reacting at least one isophthalic diester of the formula (II)

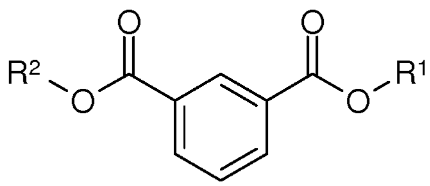

(II)

in which $R^1$ and $R^2$ are the same or different and are an aliphatic radical,
with 2 equivalents of 4-amino-2,2,6,6-tetramethylpiperidine in the presence of at least one catalyst from the group of the metal alkoxides and at a reaction temperature between 50 and 150°C.

**3.** Process according to Claim 2, **characterized in that** the metal alkoxide is a compound of the formula (VI) and/or (VII)

$X\text{-}OR^{13}$ (VI)

$R^{14}O\text{-}Y\text{-}OR^{15}$ (VII)

where

$R^{14}$ and $R^{15}$ are each the same or different and
$R^{13}$, $R^{14}$ and $R^{15}$ are aliphatic radicals each having 1 to 20 carbon atoms,
X is an alkali metal, and
Y is an alkaline earth metal.

**4.** Process according to Claim 2 or 3, **characterized in that** the metal alkoxide is sodium methoxide, potassium methoxide, lithium methoxide, sodium ethoxide, potassium ethoxide, lithium ethoxide, sodium n-propoxide, potassium n-propoxide, sodium isopropoxide, potassium isopropoxide, sodium butoxide, potassium butoxide, sodium isobutoxide, potassium isobutoxide, sodium sec-butoxide, potassium sec-butoxide, sodium tert-amoxide, sodium amoxide, potassium amoxide and/or potassium tert-butoxide.

**5.** Process according to one or more of Claims 2 to 4, **characterized in that** the metal alkoxide is added to the reaction mixture in amounts of 1 to 20 mol%, based on isophthalic diester.

**6.** Process according to one or more of Claims 2 to 5, **characterized in that** the reaction is conducted in a solvent-free manner and in a reaction apparatus from the group of the mixers, kneaders and extruders.

**7.** Process according to Claim 6, **characterized in that** the reaction mixture, after the addition of the catalyst, is mixed by the kneader, mixer or extruder at the reaction temperature for a period of 10 to 400 minutes.

**8.** Process according to Claim 6 or 7, **characterized in that** the kneader or mixer is equipped with sigma blades, masticator blades, plowshares, Becker blades and/or throwing paddles.

**9.** Process according to at least one of Claims 2 to 5, **characterized in that** the isophthalic diester is dissolved in an excess of 4-amino-2,2,6,6-tetramethylpiperidine.

**10.** Process according to at least one of Claims 2 to 5, **characterized in that** the isophthalic diester is dissolved together with about two equivalents of 4-amino-2,2,6,6-tetramethylpiperidine and a lipophilic liquid hydrocarbon or a mixture of such hydrocarbons.

**11.** Process according to one or more of Claims 2 to 10, **characterized in that** the alcohol formed in the reaction is removed from the reaction mixture.

**12.** Process according to one or more of Claims 2 to 11, **characterized in that** the reaction product of the formula (I) precipitates out of the reaction mixture in solid form.

**13.** Use of a compound of the formula (I) according to Claim 1 as a polymer additive.

**14.** Use according to Claim 13 for stabilization of polymers against light and heat and for improving the oxygen barrier properties of polymers.

**15.** Use according to Claim 13 or 14, **characterized in that** the polymers are polyamides, polyesters or EVOH.

## Revendications

**1.** Composé de formule (I)

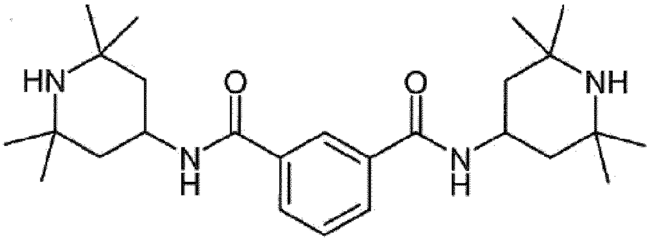

(I)

**caractérisé par** les signaux caractéristiques sur le diagramme de rayons X sur poudre, mesurés avec un rayonnement Cu $K_{alpha}$ à un angle 2-thêta de 15,0 et 22,7, qui ont une intensité forte, et de 5,0, 11,3, 18,9, 20,8, 21,6 et 23,6, qui ont une intensité moyenne.

2. Procédé de fabrication du composé de formule (I) selon la revendication 1, par mise en réaction d'au moins un diester de l'acide isophtalique de formule (II)

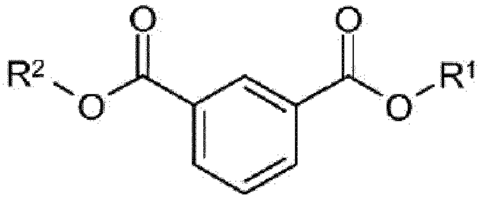

(II)

dans laquelle $R^1$ et $R^2$ sont identiques ou différents, et représentent un radical aliphatique,
avec 2 équivalents de 4-amino-2,2,6,6-tétraméthylpipéridine en présence d'au moins un catalyseur du groupe des alcoolates de métaux et à une température de réaction comprise entre 50 et 150 °C.

3. Procédé selon la revendication 2, **caractérisé en ce que** l'alcoolate de métal est un composé de la formule générale (VI) et/ou (VII) :

$X\text{-}OR^{13}$ (VI)

$R^{14}O\text{-}Y\text{-}OR^{15}$ (VII)

dans lesquelles

$R^{14}$ et $R^{15}$ sont chacun identiques ou différents, et
$R^{13}$, $R^{14}$ et $R^{15}$ représentent des radicaux aliphatiques contenant chacun 1 à 20 atomes C,
X représente un métal alcalin et
Y représente un métal alcalino-terreux.

4. Procédé selon la revendication 2 ou 3, **caractérisé en ce que** l'alcoolate de métal est le méthanolate de Na, le méthanolate de K, le méthanolate de Li, l'éthanolate de Na, l'éthanolate de K, l'éthanolate de Li, le n-propanolate de Na, le n-propanolate de K, l'isopropanolate de Na, l'isopropanolate de K, le butylate de Na, le butylate de K, l'isobutylate de Na, l'isobutylate de K, le sec.-butylate de Na, le sec.-butylate de K, le tert.-amylate de Na, l'amylate de Na, l'amylate de K et/ou le tert.-butylate de K.

5. Procédé selon une ou plusieurs des revendications 2 à 4, **caractérisé en ce que** l'alcoolate de métal est ajouté au mélange réactionnel en quantités de 1 à 20 % en moles, par rapport au diester de l'acide isophtalique.

6. Procédé selon une ou plusieurs des revendications 2 à 5, **caractérisé en ce que** la réaction est réalisée sans solvant et dans un appareil de réaction du groupe constitué par les mélangeurs, les malaxeurs et les extrudeuses.

7. Procédé selon la revendication 6, **caractérisé en ce que** le mélange réactionnel est mélangé par le malaxeur, le mélangeur ou l'extrudeuse après l'ajout du catalyseur pendant une durée de 10 à 400 minutes à la température de réaction.

8. Procédé selon la revendication 6 ou 7, **caractérisé en ce que** le malaxeur ou le mélangeur est équipé de pales sigma, de pales de masticateur, de socs de charrue, de pelles et/ou de pales d'éjection.

9. Procédé selon au moins l'une quelconque des revendications 2 à 5, **caractérisé en ce que** le diester de l'acide isophtalique est dissous dans un excès de 4-amino-2,2,6,6-tétraméthylpipéridine.

10. Procédé selon au moins l'une quelconque des revendications 2 à 5, **caractérisé en ce que** le diester de l'acide isophtalique est dissous avec environ deux équivalents de 4-amino-2,2,6,6-tétraméthylpipéridine et un hydrocarbure liquide lipophile ou un mélange de tels hydrocarbures.

11. Procédé selon une ou plusieurs des revendications 2 à 10, **caractérisé en ce que** l'alcool formé lors de la réaction est éliminé du mélange réactionnel.

12. Procédé selon une ou plusieurs des revendications 2 à 11, **caractérisé en ce que** le produit de réaction de formule (I) précipite sous forme solide à partir du mélange réactionnel.

13. Utilisation d'un composé de formule (I) selon la revendication 1 en tant qu'additif de polymère.

14. Utilisation selon la revendication 13 pour la stabilisation de polymères contre la lumière et la chaleur et pour l'amélioration des propriétés de barrière à l'oxygène de polymères.

15. Utilisation selon la revendication 13 ou 14, **caractérisée en ce que** les polymères sont des polyamides, des polyesters ou l'EVOH.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9743335 A
- WO 2004016591 A
- DE 60315329 T2